# FASCICULE DE BREVET EUROPEEN

(11) **EP 2 042 497 B1**
(45) Date de publication et mention de la délivrance du brevet: **10.03.2010**
(21) Numéro de dépôt: 08290906.0
(22) Date de dépôt: 25.09.2008
(51) Int. Cl.: C07D 333/38

(54) **Procédé de synthèse du ranélate de strontium et de ses hydrates**
Verfahren zur Synthese von Strontiumranelat und seinen Hydraten
Method for producing strontium ranelate and its hydrates

(30) Priorité: 26.09.2007 FR 0706731
(43) Date de publication de la demande: 01.04.2009
(73) Titulaire: Les Laboratoires Servier, 92415 Courbevoie Cedex (FR)
(72) Inventeur: Vaysse-Ludot, Lucile, 75490 Saint-Wandrille-Randon (FR); Lecouve, Jean-Pierre, 76600 Le Havre (FR); Langlois, Pascal, 76210 Saint-Jean-de-la-Neuville (FR)
(74) Mandataire: Giudicelli, Cathy

(56) Documents cités:
- EP-A1- 0 415 850
- EP-A1- 1 403 266
- WO-A-2007/020527
- DATABASE CA [Online] CHEMICAL ABSTRACTS SERVICE, COLUMBUS, OHIO, US; HUANG, HANZHONG ET AL: "Method for preparing strontium ranelate heptahydrate" XP002482496 extrait de STN Database accession no. 2008:111403 -& CN 101 108 845 A (TIANJIN INSTITUTE OF PHARMACEUTICAL RESEARCH, PEOP. REP. CHINA) 23 janvier 2008 (2008-01-23)

## Description

La présente invention concerne un procédé de synthèse du ranélate de strontium de formule (I) : 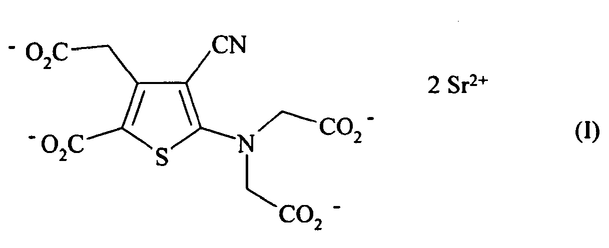 ou sel distrontique de l'acide 5-[bis(carboxyméthyl)amino]-3-carboxyméthyl-4-cyano-2-thiophènecarboxylique, et de ses hydrates.

Le ranélate de strontium possède des propriétés pharmacologiques et thérapeutiques très intéressantes, notamment des propriétés anti-ostéoporotiques remarquables, qui rendent ce composé utile dans le traitement des maladies osseuses.

Le ranélate de strontium, sa préparation et son utilisation en thérapeutique ont été décrits dans le brevet européen EP 0415 850.

Le brevet EP 0415 850 décrit trois méthodes pour la synthèse du ranélate de strontium. La deuxième des méthodes décrites consiste à porter à reflux le tétraester éthylique de formule (IIa) : 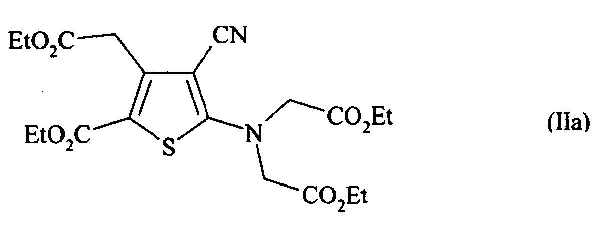 avec de la soude, en milieu hydroalcoolique, puis à distiller l'éthanol et la majorité de l'eau pour isoler le sel tétrasodique de formule (IIIa) par précipitation :

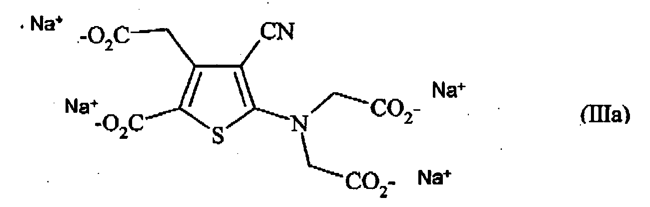

Le composé de formule (IIIa) est ensuite mis en réaction avec du chlorure de strontium, dans l'eau, pour conduire au ranélate de strontium, qui est isolé par filtration. Cependant, en opérant dans les conditions décrites pour cette deuxième méthode, la Demanderesse n'a obtenu le ranélate de strontium qu'avec un rendement inférieur à 70%.

WO 2007/020527 décrit un procédé de préparation de ranélate de strontium par réaction du tétraester éthylique de formule (IIa) avec de l'hydroxyde de lithium, suivie de la réaction du sel de lithium ainsi obtenu avec du chlorure de strontium en milieu aqueux.
EP 1 403 266 décrit un procédé de préparation de ranélate de strontium par réaction du tétraester méthylique avec l'hydroxyde de strontium.

La Demanderesse a mis au point un procédé de synthèse industriel permettant l'obtention du ranélate de strontium avec un rendement et une pureté excellents.
Plus spécifiquement, la présente invention concerne un procédé de synthèse du ranélate de strontium de formule (I) à partir d'un composé de formule (II) : 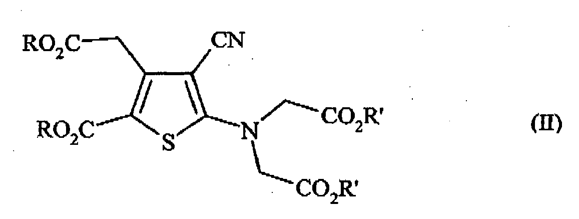 dans laquelle R et R', identiques ou différents, représentent chacun un groupement alkyle (C₁-C₆) linéaire ou ramifié, de préférence un groupement méthyle,
qui est mis en réaction avec de la soude ou de la potasse,
dans l'eau ou dans un mélange d'eau et d'un solvant organique,
à une température comprise entre 0 et 100°C,
pour conduire au sel de formule (III): 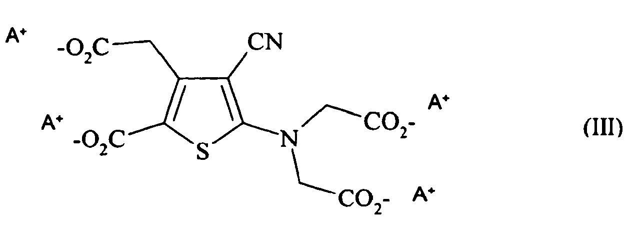 dans laquelle A représente Na ou K,
que l'on met en réaction avec du chlorure de strontium,
dans un mélange d'eau et d'un solvant organique,
à une température comprise entre 0 et 100°C,
pour conduire après isolement au ranélate de strontium ou à l'un de ses hydrates.

Parmi les solvants organiques, on peut citer à titre d'exemple le tétrahydrofurane, l'acétone, le 2-méthyl-tétrahydrofurane, le diméthylsulfoxyde, l'acétonitrile, la N-méthylpyrrolidone, les solvants alcooliques tels que le méthanol, l'éthanol, l'isopropanol, l'isobutanol.

De façon surprenante, la présence d'un solvant organique dans l'étape de salification par le chlorure de strontium permet d'augmenter le rendement de façon très significative.

La quantité de soude ou de potasse est de préférence supérieure ou égale à 4 moles par mole de composé de formule (II).

La température de la réaction de saponification est préférentiellement comprise entre 20 et 70°C.

Selon un mode de réalisation de la présente invention, le sel de formule (III) est isolé avant réaction avec le chlorure de strontium.

Selon un autre mode de réalisation, la solution du sel de formule (III) est clarifiée avant d'être engagée dans la réaction avec le chlorure de strontium.

Selon un autre mode de réalisation, la solution du sel de formule (III) est engagée telle quelle dans la réaction avec le chlorure de strontium.

La quantité de chlorure de strontium est de préférence supérieure ou égale à 2 moles par mole de composé de formule (II).

La température de la réaction avec le chlorure de strontium est préférentiellement comprise entre 20 et 50°C.

Dans le procédé selon l'invention, le ranélate de strontium est préférentiellement isolé par filtration. L'isolement par filtration du ranélate de strontium est préférentiellement suivi d'une ou plusieurs étapes de lavage par l'eau, par un solvant organique ou par un mélange eau / solvant organique, et d'une étape de séchage.

Le sel de potassium de formule (IIIb), cas particulier des composés de formule (III) pour lequel A représente K : 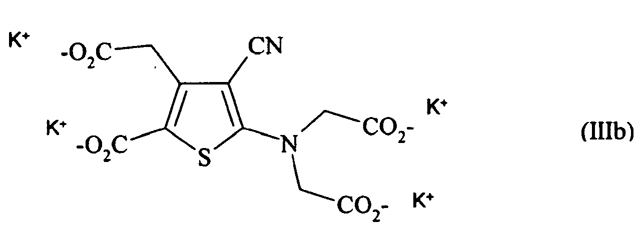 est un produit nouveau, utile comme intermédiaire de synthèse dans l'industrie chimique ou pharmaceutique, notamment dans la synthèse du ranélate de strontium et de ses hydrates, et fait à ce titre partie intégrante de la présente invention.

Les exemples ci-dessous illustrent l'invention.

### EXEMPLE 1 : Sel distrontique de l'acide 5-[bis(carboxyméthyl)amino]-3-carboxyméthyl-4-cyano-2-thiophènecarboxylique

Dans un réacteur de 800 ml muni d'une sonde de température et d'un impeller, charger à 20-25°C 50 g de 5-[bis(2-méthoxy-2-oxoéthyl)-amino]-4-cyano-3-(2-méthoxy-2-oxoéthyl)-2-thiophènecarboxylate de méthyle et 75 ml de tétrahydrofurane.
Mettre sous agitation puis charger dans le réacteur une solution aqueuse de soude préalablement préparée avec 22,9 g de NaOH et 216 ml d'eau.
Maintenir le milieu réactionnel sous agitation entre 4h et 6h.
Ajouter une solution aqueuse de chlorure de strontium préalablement préparée avec 73,9 g de SrCl₂ et 340 ml d'eau.
Maintenir sous agitation pendant 20 h entre 20 et 25°C.
La suspension s'intensifie lentement (fine suspension jaune).
Filtrer sur fritté n°3 diamètre 100 mm (filtration très rapide), et laver directement sur le fritté avec 2x 50 ml d'eau.
Expurger le produit pendant 30 min sous vide. Sécher en étuve ventilée à 30°C.

Le ranélate de strontium est ainsi obtenu avec un rendement de 93,8%.

### EXEMPLE 2 : Sel distrontique de l'acide 5-[bis(carboxyméthyl)amino]-3-carboxyméthyl-4-cyano-2-thiophènecarboxylique.

Le procédé utilisé est celui décrit dans l'Exemple 1, dans lequel on a remplacé le tétrahydrofurane par l'acétone.

Le ranélate de strontium est ainsi obtenu avec un rendement de 92,6%.

### EXEMPLE 3 : Sel distrontique de l'acide 5-[bis(carboxyméthyl)amino]-3-carboxyméthyl-4-cyano-2-thiophènecarboxylique

Le procédé utilisé est celui décrit dans l'Exemple 1, dans lequel on a remplacé la soude par 32,1 g de KOH.

La solution du sel de potassium de formule (IIIb) est clarifiée avant la réaction avec le chlorure de strontium.

Le ranélate de strontium est ainsi obtenu avec un rendement de 94 %.

### EXEMPLE 4 : Sel distrontique de l'acide 5-[bis(carboxyméthyl)amino]-3-carboxyméthyl-4-cyano-2-thiophènecarboxylique

Le procédé utilisé est celui décrit dans l'Exemple 1, dans lequel on a remplacé le tétrahydrofurane par l'isopropanol.

Le ranélate de strontium est ainsi obtenu avec un rendement de 94,8%.

### EXEMPLE 5 : Sel distrontique de l'acide 5-[bis(carboxyméthyl)amino]-3-carboxyméthyl-4-cyano-2-thiophènecarboxylique

Dans un réacteur de 800 ml muni d'une sonde de température et d'un impeller, charger à 20-25°C 22,9 g de NaOH et 500 ml d'eau. Mettre sous agitation et charger dans le réacteur 50 g de 5-[bis(2-méthoxy-2-oxoéthyl)-amino]-4-cyano-3-(2-méthoxy-2-oxoéthyl)-2-thiophènecarboxylate de méthyle .
Chauffer le milieu à 70°C en 30 min et maintenir à cette température pendant 25 min, puis clarifier à 70°C sur fritté diamètre 75 mm porosité 4 et rincer par 50 ml d'eau.
Recharger le filtrat précédent limpide orange dans un réacteur de 800 ml.
Refroidir le milieu réactionnel à 20°C et ajouter 75 ml d'éthanol, puis, en 15 minutes, une solution préalablement préparée de 73,9 g de SrCl₂ dans 137 ml d'eau.
Maintenir sous agitation pendant 2 h entre 20 et 25°C.
La suspension s'intensifie lentement (fine suspension jaune).
Filtrer sur fritté n°3 diamètre 100 mm (filtration instantanée), et réempâter directement sur le fritté avec 2x 250 ml d'eau. Sécher en étuve ventilée à 30°C.

Le ranélate de strontium est ainsi obtenu avec un rendement de 94 %.

### EXEMPLE 6 : Sel distrontique de l'acide 5-[bis(carboxyméthyl)amino]-3-carboxyméthyl-4-cyano-2-thiophènecarboxylique

### Stade A : Sel de potassium de l'acide 5-[bis(carboxyméthyl)amino]-3-carboxyméthyl-4-cyano-2-thiophènecarboxylique (composé de formule (IIIb))

Dans un réacteur de 800 ml muni d'une sonde de température et d'un impeller, charger à 20-25°C 40,4 g de KOH et 225 ml d'eau.
Mettre sous agitation puis charger dans le réacteur 60 g de 5-[bis(2-méthoxy-2-oxoéthyl)-amino]-4-cyano-3-(2-méthoxy-2-oxoéthyl)-2-thiophènecarboxylate de méthyle et 50 ml d'eau.
Chauffer le milieu réactionnel à 55-60°C en 30 min et le maintenir sous agitation pendant 2h. Effectuer une clarification à 60°C, puis refroidir le milieu réactionnel à 20-25°C.
Mettre à sec sous vide à 40°C Ajouter sur le résidu ainsi obtenu 200 ml d'acétate d'éthyle et 20 ml de méthanol. Agiter pendant 8 h. Filtrer la suspension obtenue. Expurger le produit pendant 30 min sous vide. Sécher en étuve ventilée à 30°C.

### Stade B : Sel distrontique de l'acide 5-[bis(carboxyméthyl)amino]-3-carboxyméthyl-4-cyano-2-thiophènecarboxylique

A 62,0 g du sel de potassium obtenu au stade précédent, ajouter 75 ml de tétrahydrofurane et 216 ml d'eau, puis une solution aqueuse de chlorure de strontium préalablement préparée avec 73,9 g de SrCl₂ et 340 ml d'eau.
Maintenir sous agitation pendant 20 h entre 20 et 25°C.
La suspension s'intensifie lentement. Filtrer sur fritté n°3 diamètre 100 mm, et laver directement sur le fritté avec 2x 50 ml d'eau.
Expurger le produit pendant 30 min sous vide. Sécher en étuve ventilée à 30°C.

## Revendications

1. Procédé de synthèse du ranélate de strontium de formule (I) : 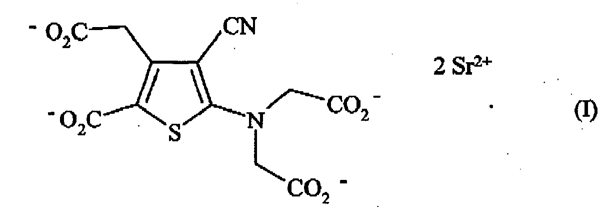 et de ses hydrates,
par réaction du composé de formule (II) : 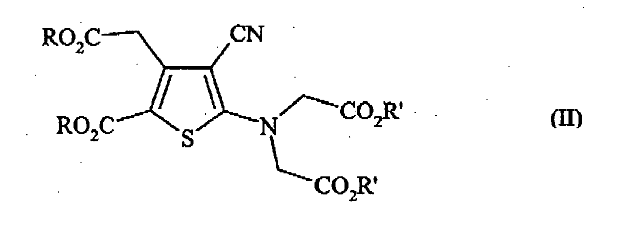 dans laquelle R et R', identiques ou différents, représentent chacun un groupement alkyle (C₁-C₆) linéaire ou ramifié,
avec de la soude ou de la potasse,
dans l'eau ou dans un mélange d'eau et d'un solvant organique choisi parmi le tétrahydrofurane, l'acétone, le 2-méthyl-tétrahydrofurane, le diméthylsulfoxyde, l'acétonitrile, la N-méthylpyrrolidone, le méthanol, l'éthanol, l'isopropanol et l'isobutanol,
à une température comprise entre 0 et 100°C,
pour conduire au sel de formule (III): 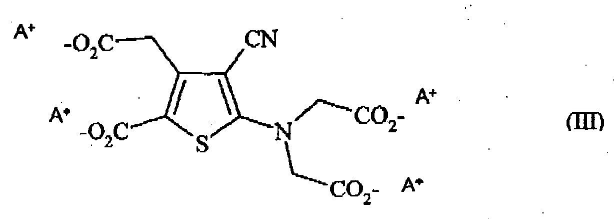 dans laquelle A représente Na ou K,
que l'on met en réaction avec du chlorure de strontium,
à une température comprise entre 0 et 100°C,
pour conduire après isolement au ranélate de strontium ou à l'un de ses hydrates,
**caractérisé en ce que** l'étape de réaction du sel de formule (III) avec le chlorure de strontium est réalisée dans un mélange d'eau et d'un solvant organique choisi parmi le tétrahydrofurane, l'acétone, le 2-méthyl-tétrahydrofurane, le diméthylsulfoxyde, l'acétonitrile, la N-méthylpyrrolidone, le méthanol, l'éthanol, l'isopropanol et l'isobutanol.

2. Procédé de synthèse selon la revendication 1, dans lequel la quantité de soude ou de potasse est supérieure ou égale à 4 moles par mole de composé de formule (II).

3. Procédé de synthèse selon, la revendication 1 ou 2, dans lequel la température de la réaction de saponification du composé de formule (II) est comprise entre 20 et 70°C.

4. Procédé de synthèse selon l'une quelconque des revendications 1 à 3, dans lequel le sel de formule (III) est isolé avant réaction avec le chlorure de strontium.

5. Procédé de synthèse selon l'une quelconque des revendications 1 à 3, dans lequel la solution du sel de formule (III) est clarifiée avant d'être engagée dans la réaction avec le chlorure de strontium.

6. Procédé de synthèse selon l'une quelconque des revendications 1 à 3, dans lequel la solution du sel de formule (III) est engagée telle quelle dans la réaction avec le chlorure de strontium.

7. Procédé de synthèse selon l'une quelconque des revendications 1 à 6, dans lequel la quantité de chlorure de strontium est supérieure ou égale à 2 moles par mole de composé de formule (II).

8. Procédé de synthèse selon l'une quelconque des revendications 1 à 7, dans lequel la température de la réaction de salification du composé de formule (III) est comprise entre 20 et 50°C.

9. Procédé de synthèse selon l'une quelconque des revendications 1 à 8, dans lequel le ranélate de strontium est isolé par filtration.

10. Procédé de synthèse selon la revendication 9, dans lequel l'étape de filtration est suivie d'une ou plusieurs étapes de lavage et d'une étape de séchage.

11. Composé de formule (IIIb) :

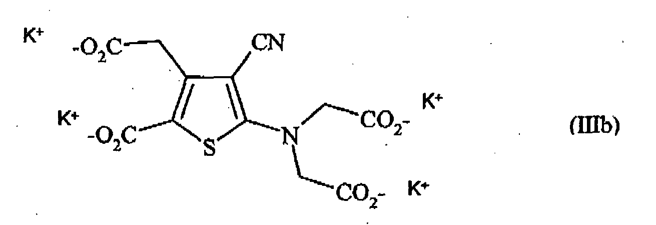

12. Procédé de synthèse selon l'une quelconque des revendications 1 à 10, dans lequel R et R' représentent chacun un groupement méthyle.

## Claims

1. Process for the synthesis of strontium ranelate of formula (I): 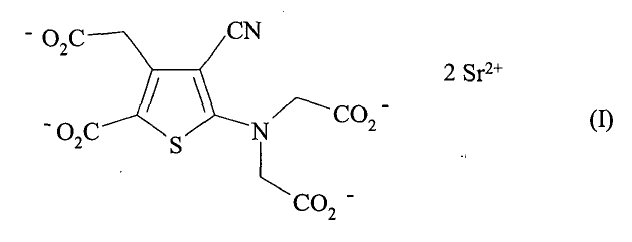 and its hydrates
by reaction of the compound of formula (II): 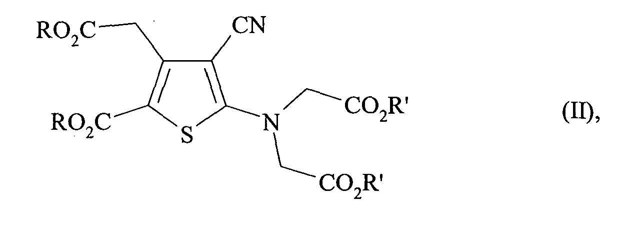 wherein R and R', which may be the same or different, each represent a linear or branched (C₁-C₆)alkyl group,
with sodium hydroxide or potassium hydroxide,
in water or in a mixture of water and an organic solvent selected from tetrahydrofuran, acetone, 2-methyl-tetrahydrofuran, dimethyl sulphoxide, acetonitrile, N-methylpyrrolidone, methanol, ethanol, isopropanol and isobutanol,
at a temperature from 0 to 100°C,
to yield the salt of formula (III): 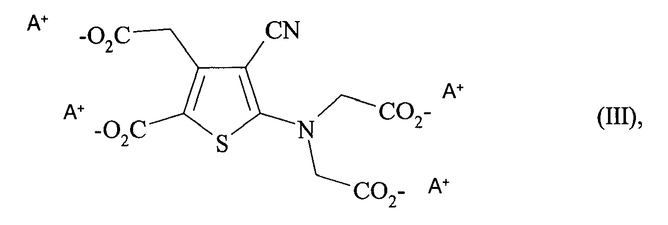 wherein A represents Na or K,
which is reacted with strontium chloride,
at a temperature from 0 to 100°C,
to yield, after isolation, strontium ranelate or one of its hydrates,
**characterised in that** the step of reacting the salt of formula (III) with strontium chloride is carried out in a mixture of water and an organic solvent selected from tetrahydrofuran, acetone, 2-methyl-tetrahydrofuran, dimethyl sulphoxide, acetonitrile, N-methylpyrrolidone, methanol, ethanol, isopropanol and isobutanol.

2. Synthesis process according to claim 1, wherein the amount of sodium hydroxide or potassium hydroxide is greater than or equal to 4 moles per mole of compound of formula (II).

3. Synthesis process according to claim 1 or 2, wherein the temperature of the saponification reaction on the compound of formula (II) is from 20 to 70°C.

4. Synthesis process according to any one of claims 1 to 3, wherein the salt of formula (III) is isolated before reaction with strontium chloride.

5. Synthesis process according to any one of claims 1 to 3, wherein the solution of the salt of formula (III) is clarified before being used in the reaction with strontium chloride.

6. Synthesis process according to any one of claims 1 to 3, wherein the solution of the salt of formula (III) is used as such in the reaction with strontium chloride.

7. Synthesis process according to any one of claims 1 to 6, wherein the amount of strontium chloride is greater than or equal to 2 moles per mole of compound of formula (II).

8. Synthesis process according to any one of claims 1 to 7, wherein the temperature of the salt conversion reaction on the compound of formula (III) is from 20 to 50°C.

9. Synthesis process according to any one of claims 1 to 8, wherein the strontium ranelate is isolated by filtration.

10. Synthesis process according to claim 9, wherein the filtration step is followed by one or more washing steps and a drying step.

11. Compound of formula (IIIb):

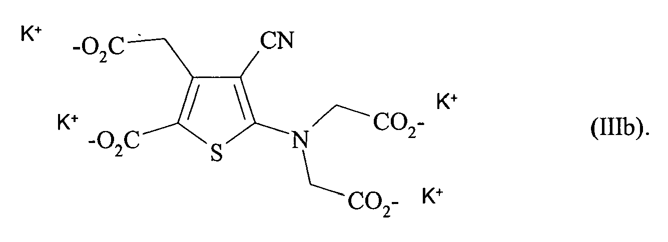

12. Synthesis process according to any one of claims 1 to 10, wherein R and R' each represent a methyl group.

## Patentansprüche

1. Verfahren zur Synthese von Strontiumranelat der Formel (I): 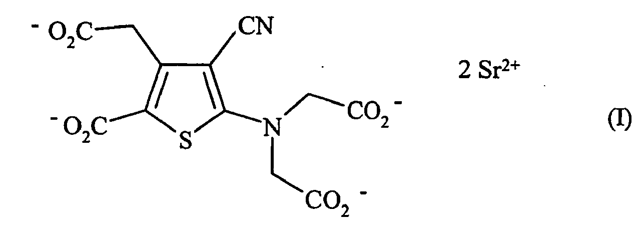 und seinen Hydraten
durch Umsetzen der Verbindung der Formel (II): 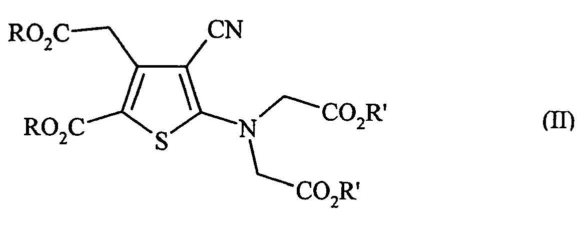 in der R und R', die identisch oder verschieden sind, jeweils eine geradkettige oder verzweigte (C₁-C₆)-Alkylgruppe bedeuten,
mit Natriumhydroxid oder Kaliumhydroxid
in Wasser oder in einer Mischung aus Wasser und einem organischen Lösungsmittel ausgewählt aus Tetrahydrofuran, Aceton, 2-Methyl-tetrahydrofuran, Dimethylsulfoxid, Acetonitril, N-Methylpyrrolidon, Methanol, Ethanol, Isopropanol und Isobutanol,
bei einer Temperatur zwischen 0 und 100 °C
zur Bildung des Salzes der Formel (III): 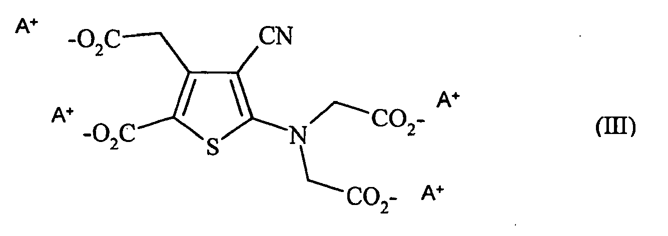 in der A Na oder K bedeutet,
welches man mit Strontiumchlorid
bei einer Temperatur zwischen 0 und 100 °C umsetzt,
sodass man nach der Isolierung Strontiumranelat oder eines seiner Hydrate erhält,
**dadurch gekennzeichnet, dass** die Stufe der Umsetzung des Salzes der Formel (III) mit Strontiumchlorid in einer Mischung aus Wasser und einem organischen Lösungsmittel ausgewählt aus Tetrahydrofuran, Aceton, 2-Methyl-tetrahydrofuran, Dimethylsulfoxid, Acetonitril, N-Methylpyrrolidon, Methanol, Ethanol, Isopropanol und Isobutanol durchgeführt wird.

2. Syntheseverfahren nach Anspruch 1, worin die Menge an Natriumhydroxid oder Kaliumhydroxid größer oder gleich ist als 4 Mol pro Mol der Verbindung der Formel (II).

3. Syntheseverfahren nach Anspruch 1 oder 2, worin die Temperatur der Verseifungsreaktion der Verbindung der Formel (II) zwischen 20 und 70 °C liegt.

4. Syntheseverfahren nach einem der Ansprüche 1 bis 3, worin das Salz der Formel (III) vor der Umsetzung mit Strontiumchlorid isoliert wird.

5. Syntheseverfahren nach einem der Ansprüche 1 bis 3, worin die Lösung des Salzes der Formel (III) geklärt wird, bevor sie bei der Reaktion mit Strontiumchlorid eingesetzt wird.

6. Syntheseverfahren nach einem der Ansprüche 1 bis 3, worin die Lösung des Salzes der Formel (III) so, wie sie ist, bei der Umsetzung mit Strontiumchlorid verwendet wird.

7. Syntheseverfahren nach einem der Ansprüche 1 bis 6, worin die Strontiumchloridmenge größer oder gleich ist als 2 Mol pro Mol der Verbindung der Formel (II).

8. Syntheseverfahren nach einem der Ansprüche 1 bis 7, worin die Temperatur der Reaktion der Salzbildung der Verbindung der Formel (III) zwischen 20 und 50 °C liegt.

9. Syntheseverfahren nach einem der Ansprüche 1 bis 8, worin Strontiumranelat durch Filtration isoliert wird.

10. Syntheseverfahren nach Anspruch 9, worin die Stufe der Filtration von einer oder mehreren Stufen des Waschens und einer Trocknungsstufe gefolgt wird.

11. Verbindung der Formel (IIIb):

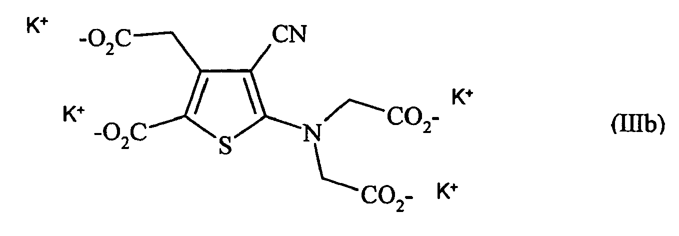

12. Syntheseverfahren nach einem der Ansprüche 1 bis 10, worin R und R' jeweils eine Methylgruppe bedeuten.
